(19) **Europäisches Patentamt**
**European Patent Office**
**Office européen des brevets**

(11) **EP 4 427 779 B1**

(12) **EUROPEAN PATENT SPECIFICATION**

(45) Date of publication and mention
of the grant of the patent:
**15.04.2026  Bulletin 2026/16**

(21) Application number: **22914705.3**

(22) Date of filing: **26.12.2022**

(51) International Patent Classification (IPC):
***A61M 16/00*** (2006.01)      ***A61B 5/08*** (2006.01)
***A61B 5/087*** (2006.01)

(52) Cooperative Patent Classification (CPC):
**A61M 16/024; A61B 5/087; A61M 16/0003;**
**A61M 16/026;** A61M 2016/0021; A61M 2016/0027;
A61M 2016/0036; A61M 2205/505

(86) International application number:
**PCT/CN2022/142089**

(87) International publication number:
**WO 2023/125451 (06.07.2023 Gazette 2023/27)**

(54) **BREATHING PHASE DETERMINATION METHOD AND APPARATUS**

VERFAHREN UND VORRICHTUNG ZUR BESTIMMUNG DER ATEMPHASE

PROCÉDÉ ET APPAREIL DE DÉTERMINATION DE LA PHASE RESPIRATOIRE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **31.12.2021  CN 202111678010**

(43) Date of publication of application:
**11.09.2024  Bulletin 2024/37**

(73) Proprietor: **BMC (Tianjin) Medical Co., Ltd.**
**Tianjin 301700 (CN)**

(72) Inventors:
• **WANG, Xiaochen**
**Tianjin 301700 (CN)**
• **ZHUANG, Zhi**
**Tianjin 301700 (CN)**
• **ZHENG, Fang**
**Tianjin 301700 (CN)**

• **TIAN, Xin**
**Tianjin 301700 (CN)**

(74) Representative: **Grünecker Patent- und**
**Rechtsanwälte**
**PartG mbB**
**Leopoldstraße 4**
**80802 München (DE)**

(56) References cited:
AU-A1- 2003 273 226      CN-A- 102 762 247
CN-A- 109 303 960       CN-A- 110 180 059
CN-A- 112 156 297       CN-A- 114 469 060
CN-U- 211 561 460       CN-U- 211 561 460
US-A- 5 572 993         US-A- 5 572 993
US-A1- 2006 241 509     US-A1- 2010 097 380
US-A1- 2018 093 055     US-A1- 2018 177 433
US-A1- 2021 379 306

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## TECHNICAL FIELD

Embodiments of the present disclosure relate to the field of medical apparatus and instruments, in particular to a method for determining a respiratory phase, an apparatus for determining a respiratory phase, an electronic device, and a readable storage medium.

## BACKGROUND

Ventilation therapy devices continuously supply oxygen through a nasal oxygen tube inserted into the nasal cavity of a patient. The patient's respiratory process is divided into two processes, that is, an inspiratory process and an expiratory process. During the inspiratory process, the patient needs to inspirate a large amount of oxygen, and no oxygen is needed during the expiratory process. Accordingly, it is necessary to identify the patient's respiratory phase (inspiratory or expiratory) in real time to adjust the oxygen supply according to the respiratory phase.

Since the nasal oxygen tube is an open airway, the gas flow and pressure values of the nasal oxygen tube collected by the device are difficult to directly reflect the patient's own respiration conditions. Therefore, an additional measurement method is usually added for the identification of the respiratory phase. A common method is to introduce a pressure measurement conduit from the nasal oxygen tube for directly measuring the pressure change inside the nasal oxygen tube, so as to determine the respiratory phase. Alternatively, the patient's respiration conditions are measured through thermal sensors, chest and abdominal belts and the like, so as to determine the respiratory phase. However, both of such two methods require the addition of additional devices and have complex structures.

US2021379306 A1 discloses a ventilation therapy apparatus and a control method, said ventilation therapy apparatus comprising an apparatus body, a respiratory pipe and a patient interface, wherein the actual pressure value and the actual flow value are measured at the signal collection point of the apparatus body.

## SUMMARY

Embodiments of the present disclosure provide a method and an apparatus for determining a respiratory phase, an electronic device and a readable storage medium, so as to solve the technical problem that the structure is complex because additional devices are required to be added to determine the respiratory phase.

In a first aspect, an embodiment of the present disclosure provides a method for determining a respiratory phase, applied to a ventilation therapy device including a respiratory conduit, and the method includes:

acquiring a gas flow value of a respiratory conduit, and acquiring a pressure value of a pressure sensor, wherein the pressure sensor is used for collecting a pressure in the respiratory conduit;
converting the gas flow value and the pressure value into a first value and a second value under a same reference;
determining a current respiratory phase of the patient based on a difference of the first value and the second value.

Optionally, the converting the gas flow value and the pressure value into a first value and a second value under a same reference includes:
converting the gas flow value and the pressure value into a first value and a second value under the same physical quantity.

Optionally, the converting the gas flow value and the pressure value into the first value and the second value under the same physical quantity includes:
converting the gas flow value into the first value under a pressure physical quantity, and using the pressure value as the second value.

Optionally, the converting the gas flow value into a first value under the pressure physical quantity includes:
obtaining the first value under the pressure physical quantity by multiplying a square of the gas flow value by a drag coefficient of the ventilation therapy device.

Optionally, the converting the gas flow value and the pressure value into the first value and the second value under the same physical quantity includes:
converting the pressure value into a second value under the flow physical quantity, and using the gas flow value as the first value.

Optionally, the converting the pressure value into a second value under the flow physical quantity includes:

the pressure value being divided by the drag coefficient of the ventilation therapy device to obtain an intermediate value;
obtaining the second value under the flow physical quantity by finding a square root of the intermediate value.

Optionally, the converting the gas flow value and the pressure value into a first value and a second value under a same reference includes:

scaling the gas flow value and using the scaled gas flow value as the first value, and using the pressure value as the second value; or
scaling the pressure value and using the scaled pressure value as the second value, and using the gas flow value as the first value.

Optionally, the determining a current respiratory phase of the patient based on a difference of the first value and the second value includes:

obtaining a first difference by subtracting the second value from the first value;
determining the current respiratory phase of the patient based on the first difference.

Optionally, the determining the current respiratory phase of the patient based on the first difference includes:

determining the respiratory phase as the inspiratory phase in response to the first difference being greater than 0;
determining the respiratory phase as the expiratory phase in response to the first difference being less than 0.

In a second aspect, an embodiment of the present disclosure provides an apparatus for determining a respiratory phase, which is applied to a ventilation therapy device including a respiratory conduit, and the apparatus includes:

an acquisition module, configured to acquire a gas flow value of a respiratory conduit, and acquire a pressure value of a pressure sensor, wherein the pressure sensor is used for collecting a pressure in the respiratory conduit;
a conversion module, configured to convert the gas flow value and the pressure value into a first value and a second value under the same reference; and
a determination module, configured to determine a current respiratory phase of the patient based on a difference of the first value and the second value.

In a third aspect, an embodiment of the present disclosure provides an electronic device, including a processor, a memory, and a program or instruction stored in the memory and executable on the processor, wherein the program or instruction implements the described method when executed by the processor.

In a fourth aspect, an embodiment of the present disclosure provides a readable storage medium storing a program or instruction that, when executed by a processor, implements the described method.

In the embodiments of the present disclosure, the pressure value and the gas flow value of the respiratory conduit are acquired, and the pressure value and the gas flow value are converted into a first value and a second value under the same reference, and the current respiratory phase of the patient is determined based on the difference between the first value and the second value. The first value and the second value are values obtained through conversion, and are values under the same reference, so a subtraction may be performed on the first value and the second value to obtain a difference related to the patient's respiration, thereby determining the patient's current respiratory phase based on the difference. In other words, the patient's respiratory phase can be determined by using only the pressure value and the gas flow value of the respiratory conduit, without adding additional auxiliary judgment device, thereby simplifying the structure of the ventilation therapy device.

**BRIEF DESCRIPTION OF THE DRAWINGS**

In order to explain technical solutions of the embodiments of the present disclosure more clearly, accompanying drawings used in the illustration of the embodiments will be briefly introduced. Apparently, the accompanying drawings in the following explanation illustrate merely some embodiments of the present disclosure, and those skilled in the art may obtain other accompanying drawings based on these accompanying drawings without paying any creative effort.

FIG. 1 is a flowchart of a method for determining a respiratory phase provided by an embodiment of the present disclosure;
FIG. 2 is a graph illustrating changes in sensor signals caused by respiration of a patient in an embodiment of the present disclosure;
FIG. 3 is a graph illustrating changes in sensor signals caused by adjustment of a ventilation therapy device in an embodiment of the present disclosure;
FIG. 4 is a diagram illustrating a relationship between the graph in FIG. 2 as well as the graph in FIG. 3 and a sensor signal graph in an embodiment of the present disclosure;
FIG. 5 is a diagram illustrating a graph of a first value, a second value, and a graph of the difference in an embodiment of the present disclosure;
FIG. 6 is a comparison diagram of the sensor signal diagram before and after filtering in an embodiment of the present disclosure;
FIG.7 is a first schematic diagram of an electronic device provided in an embodiment of the present disclosure; and
FIG. 8 is a second schematic diagram of an electronic device provided in an embodiment of the present disclosure.

**DETAILED DESCRIPTION OF THE EMBODIMENTS**

A clear and thorough description for technical solutions in the embodiments of the present disclosure will be given below in conjunction with the accompanying drawings in the embodiments of the present disclosure. Apparently, the described embodiments are a part of embodiments of the present disclosure, not all the embodiments.

The method and apparatus for determining a respiratory phase, an electronic device and a readable storage medium provided in the embodiments of the present disclosure are described in detail below with reference to the accompanying drawings.

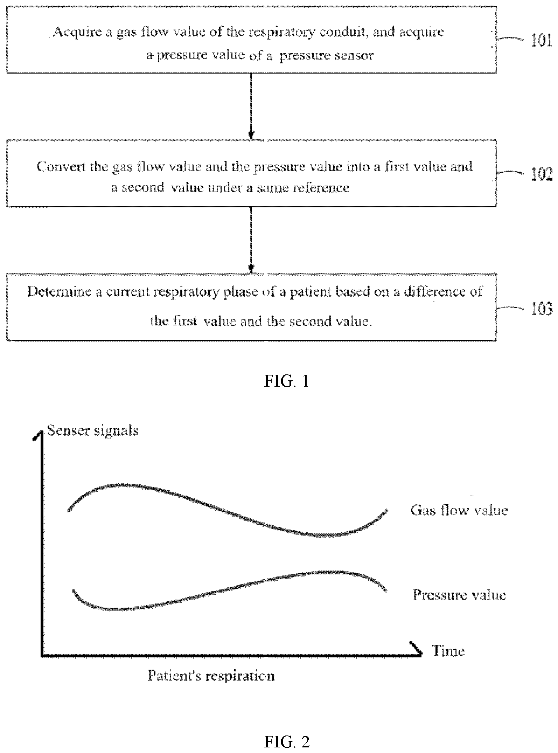

In a first aspect, an embodiment of the present disclosure provides a method for determining a respiratory phase, which is applied to a ventilation therapy device. As shown in FIG. 1, the method includes steps described below.

At step 101, a gas flow value of a respiratory conduit is acquired, and a pressure value of a pressure sensor is acquired. The pressure sensor is used for collecting the pressure in the respiratory conduit.

The ventilation therapy device in the embodiments of the present disclosure may include a device body, a respiratory conduit, a patient interface, a flow sensor and a pressure sensor. The device body is configured to output gas with a preset pressure and a preset flow, and the device body includes a gas outlet. The respiratory conduit includes a first end and a second end that communicate with each other, the first end of the respiratory conduit being connected to the gas outlet, and the second end being connected to the patient interface. The patient interface is used to be worn at the patient's nasal cavity, and when the patient interface is worn at the patient's nasal cavity, an air outlet gap is provided between the patient interface and the patient's nasal cavity. The pressure sensor is configured to acquire the pressure value of the respiratory conduit, and the flow sensor is configured to acquire the gas flow value of the respiratory conduit.

In specific applications, a flow sensor may be used to acquire the gas flow value of the respiratory conduit, and a pressure sensor may be used to acquire the pressure value of the respiratory conduit.

At step 102, the gas flow value and the pressure value are converted into a first value and a second value under the same reference.

The pressure value and gas flow value in the respiratory conduit may change when the patient breathes. Similarly, the pressure value and gas flow value in the respiratory conduit may change when the output pressure and output flow of the ventilation therapy device are adjusted. In other words, the obtained pressure value and gas flow value are a cooperation result of the patient's respiration and the ventilation therapy device.

As shown in FIG. 2, when the patient inhales, a large amount of gas is inhaled, which causes the pressure value of the respiratory conduit to decrease and the gas flow value to increase; when the patient exhales, the pressure value of the respiratory conduit will increase and the gas flow value will decrease. That is, the changes in the pressure value and the gas flow value caused by the patient's respiration are inverse. In other words, if the pressure value decreases, then the gas flow value increases; if the pressure value increases, then the gas flow value decreases.

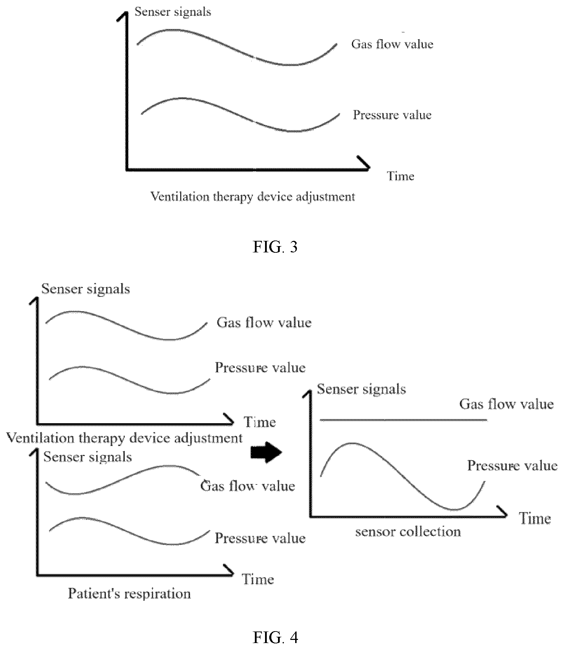

As shown in FIG. 3, when the output flow of the ventilation therapy device increases, the obtained pressure value of the respiratory conduit increases, and the obtained gas flow value increases; when the output flow of the ventilation therapy device decreases, the obtained pressure value of the respiratory conduit decreases, and the obtained gas flow value decreases. That is, the changes in the pressure value and the gas flow value caused by the ventilation therapy device are in the same direction. In other words, if the pressure value decreases, then the gas flow value decreases; if the pressure value increases, then the gas flow value increases.

The pressure value and gas flow value may be expressed as follows through formulas: pressure value = change in pressure value caused by patient's respiration + change in pressure value caused by the ventilation therapy device; gas flow value = change in gas flow value caused by patient's respiration + change in gas flow value caused by the ventilation therapy device. Assuming that an increase in pressure value or gas flow value is defined as positive, and a decrease in pressure value or gas flow value is defined as negative, since the changes in the pressure value and the gas flow value caused by the patient's respiration are inverse, the signs for the factor of patient's respiration in the two formulas are inverse; while the changes in the pressure value and the gas flow value caused by the ventilation therapy device are in the same direction, the signs for the factor of the ventilation therapy device in the two formulas are the same. Therefore, the changes caused by the factor of the ventilation therapy device may be eliminated by subtracting the two formulas.

Since the pressure value and the gas flow value are physical quantities of different dimensions and their numerical values are also different, the two formulas cannot be directly subtracted and a conversion is required. The gas flow value and the pressure value are converted to the first value and the second value under the same reference.

The same reference may mean that the first value and the second value belong to physical quantities of the same dimension, or may mean that an average of the first value and the second value is within a predetermined range.

In specific applications, the pressure value may be used as a reference, and the gas flow value is converted to a value with the same reference as the pressure value. Alternatively, the gas flow value may be used as a reference, and the pressure value is converted to a value with the same reference as the gas flow value. Alternatively, the pressure value and the gas flow value may also be converted to another same reference.

At step 103, a current respiratory phase of the patient is determined based on a difference between the first value and the second value.

As can be seen from the above formulas, a difference obtained through subtraction of the converted first value and the second value is only related to the change caused by the factor of patient's respiration, so the current respiratory phase of the patient can be determined based on the difference.

In the embodiment of the present disclosure, the pressure value and the gas flow value of the respiratory conduit are acquired, and the pressure value and the gas flow value are converted into a first value and a second value under the same reference, and the current respiratory phase of the patient is determined based on the difference between the first value and the second value. The first value and the second value are values obtained through conversion, and are values under the same reference, so a subtraction maybe performed on the first value and the second value to obtain a difference related to the factor of the patient's respiration, thereby determining the patient's current respiratory phase based on the difference. In other words, the patient's respiratory phase can be determined by using only the pressure value and the gas flow value of the respiratory conduit, without adding additional auxiliary judgment device, thereby simplifying the structure of the ventilation therapy device.

Optionally, in an embodiment of the present disclosure, step 102, in which the gas flow value and the pressure value are converted into a first value and a second value under the same reference, includes:
converting the gas flow value and the pressure value into a first value and a second value under the same physical quantity.

Generally speaking, the gas flow value and the pressure value in the gas circuit satisfy the following formula:

$$P = \mu \times F^2$$

among them, P is the pressure value; F is the gas flow value; $\mu$ is a drag coefficient, which is an inherent parameter of the ventilation therapy device, and can be measured in advance through experiments or be iteratively calculated during operation.

Thus, it can be known that the obtained pressure value and gas flow value can be converted through the above formula to obtain a first value and a second value of the same physical quantity, so as to perform a subtraction on the first value and the second value to obtain a difference related to the patient's respiration.

Optionally, in an embodiment of the present disclosure, the converting the gas flow value and the pressure value into a first value and a second value of the same physical quantity includes:
converting the pressure value into a second value under the flow physical quantity, and using the gas flow value as the first value.

The obtained pressure value is converted through the above formula to obtain a second value under the flow physical quantity, so as to perform the subtraction on the first value and the second value to obtain a difference value related to the patient's respiration.

Further, the converting the pressure value into a second value under the flow physical quantity includes:

the pressure value being divided by the drag coefficient of the ventilation therapy device to obtain an intermediate value; and
obtaining the second value under the flow physical quantity by finding the square root of the intermediate value.

In specific applications, the pressure value is divided by the drag coefficient to obtain an intermediate value, which is the square of the gas flow value. The square root of the intermediate value is then found to obtain the second value under the flow physical quantity.

The obtained pressure value is converted through the above formula to obtain a second value under the flow physical quantity, so as to perform the subtraction on the first value and the second value to obtain a difference value related to the patient's respiration.

Optionally, in an embodiment of the present disclosure, the converting the gas flow value and the pressure value into a first value and a second value under the same physical quantity includes:
converting the gas flow value into a first value under the pressure physical quantity, and using the pressure value as the second value.

The obtained gas flow value is converted through the above formula to obtain a first value under the pressure physical quantity, so as to perform the subtraction on the first value and the second value to obtain a difference related to the patient's respiration.

Optionally, in an embodiment of the present disclosure, the converting the gas flow value into a first value under the pressure physical quantity includes:
obtaining a first value under the pressure physical quantity by multiplying the square of the pressure value by the drag coefficient of the ventilation therapy device.

The obtained pressure value is converted through the above formula to obtain a second value under the flow physical quantity, so as to perform the subtraction on the first value and the second value to obtain a difference value related to the patient's respiration.

It should be noted that in the above embodiments, it is not necessary to calculate the precise values when converting the gas flow value into the pressure value and vice versa, as long as the calculated first value and second value can reflect an approximate variation rule of the values. For example, when converting the gas flow value into a pressure value, the pressure value P converted from the gas flow value = the square of the change in the gas flow value caused by the patient's respiration+ the square of the change in the gas flow value caused by the ventilation therapy device $\pm$ 2 * the change in the gas flow value caused by the patient's respiration * the change in the gas flow value caused by the ventilation therapy device. However, in the embodiment of the present disclosure, an approximate number is used, that is, the pressure value P converted from the gas flow value = the square of the change in the gas flow value caused by the patient's respiration +the square of the change in the gas flow value caused by the ventilation therapy device, which can reduce the difficulty of calculation.

Optionally, in an embodiment of the present disclosure, the gas flow value and the pressure value are converted into a first value and a second value under the same reference by:

scaling the gas flow value and using the scaled gas flow value as the first value, and using the pressure value as the second value; or
scaling the pressure value and using the scaled pressure value as the second value, and using the gas flow value as the first value.

In specific applications, the magnification ratio and reduction ratio may be flexibly selected according to actual conditions. For example, the gas flow value and pressure value collected during at least one respiratory cycle of the patient are acquired, the gas flow value is averaged to obtain a first mean, and the pressure value is averaged to obtain a second mean. The magnification ratio and reduction ratio may be a ratio of the first mean to the second mean.

After the gas flow value or the pressure value is scaled, the gas flow value and the pressure value are under the same reference, so a subtraction is performed on the first value and the second value to obtain a difference related to the patient's respiration, thereby determining the respiratory phase of the patient based on the difference.

Optionally, in an embodiment of the present disclosure, a current respiratory phase of the patient is determined based on a difference between the first value and the second value by:

obtaining a first difference by subtracting the second value from the first value; and
determining the current respiratory phase of the patient based on the first difference.

Since the changes in the pressure value and the gas flow value caused by the patient's respiration are in inverse trends, and the changes in the pressure value and the gas flow value caused by the ventilation therapy device are in the same trend, the changes caused by the ventilation therapy device can be eliminated by subtracting the second value from the first value to obtain the first difference related to the patient's respiration, and the patient's respiration phase may be determined based on the first difference.

Optionally, in an embodiment of the present disclosure, the determining the current respiratory phase of the patient based on the first difference includes:

determining the respiratory phase as the inspiratory phase in response to the first difference being greater than 0;

determining the respiratory phase as the expiratory phase in response to the first difference being less than 0.

It should be noted that, in a procedure of processing the pressure value and the gas flow value in the above embodiment, a pressure value change curve and a gas flow value change curve can be obtained respectively according to the pressure value and the gas flow value, which are converted to obtain a first value change curve and a second value change curve, and a difference change curve is obtained according to the first value change curve and the second value change curve, and then the respiratory phase of the patient is determined based on the difference change curve.

For ease of understanding, the following description will be given assuming that the ventilation therapy device is in a constant flow mode. As shown in FIG. 4, the upper left curve graph of FIG. 4 is a curve graph illustrating the changes in pressure value and gas flow value in the respiratory conduit caused by adjusting the output flow and output pressure of the ventilation therapy device, the lower left curve graph of FIG. 4 is a curve graph illustrating the changes in pressure value and gas flow value in the respiratory conduit caused by the patient's respiration, and the right graph of FIG. 4 illustrates a gas flow value curve and a pressure value curve of the respiratory conduit obtained by devices such as sensors.

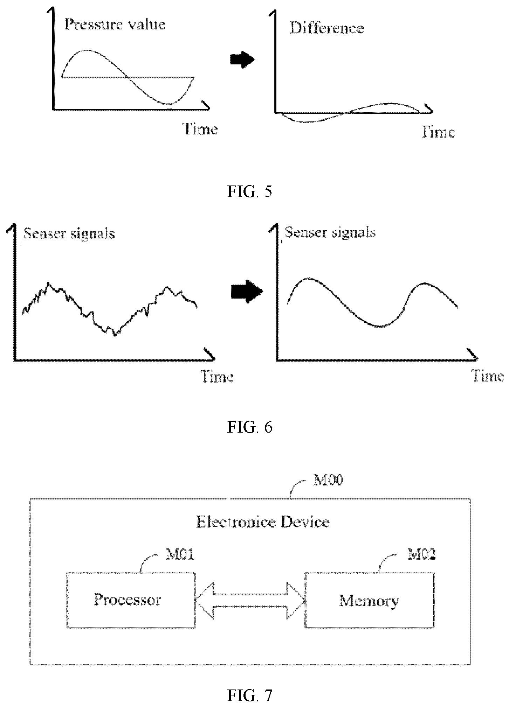

After converting the gas flow value into a pressure value, a curve graph as shown on the left side of FIG. 5 is obtained, in which the straight line is the first value obtained through conversion, and the curved line is the second value. A difference curve on the right side of FIG. 5 is obtained by performing a subtraction on the two curves on the left side of FIG. 5, that is, subtracting the first value from the second value. The respiratory phase of the patient is determined based on the difference curve.

A part of the difference curve over the horizontal axis is the inspiratory phase, and a part of the difference curve below the horizontal axis is the expiratory phase.

When the gas flow value and the pressure value are acquired, since the signal-to-noise ratio of the collected signal is relatively low, high-frequency noise is it generally contained, and the frequency of the respiratory signal that we are concerned about is generally lower than 1 Hz. Therefore, as shown in FIG. 6, the signal may be filtered first, and a low-pass filter may be used to filter out the high-frequency signal to obtain a cleaner signal.

In a second aspect, an embodiment of the present disclosure provides an apparatus for determining a respiratory phase, which is applied to a ventilation therapy device including a respiratory conduit, and the apparatus includes:

an acquisition module, configured to acquire a gas flow value of a respiratory conduit, and acquire a pressure value of a pressure sensor that is used to collect the pressure in the respiratory conduit;

a conversion module, configured to convert the gas flow value and the pressure value into a first value and a second value under the same reference; and

a determination module, configured to determine a current respiratory phase of the patient based on a difference of the first value and the second value.

In the embodiment of the present disclosure, the pressure value and gas flow value of the respiratory conduit are acquired, and the pressure value and the gas flow value are converted into a first value and a second value under the same reference, thereby determining the current respiratory phase of the patient based on the difference of the first value and the second value. The first value and the second value are obtained through conversion, and are values under the same reference. Therefore, the subtraction may be performed on the first value and the second value to obtain a difference related to the factor of the patient's respiration, thereby determining the current respiratory phase of the patient based on the difference. In other words, the patient's respiratory phase can be determined by using only the pressure value and gas flow value of the respiratory conduit without adding additional auxiliary judgment device, thus simplifying the structure of the ventilation therapy device.

The apparatus in the embodiment of the present disclosure may be a device, or a component, integrated circuit, or chip in a terminal. The device may be a mobile electronic device, or a non-mobile electronic device. Exemplarily, the mobile electronic device may be a mobile phone, a tablet computer, a laptop computer, a PDA, an in-vehicle electronic device, a wearable device, an ultra-mobile personal computer (UMPC), a netbook, or a personal digital assistant (PDA), etc., and the non-mobile electronic device may be a server, a network attached storage (NAS), a personal computer (PC), a television (TV), a teller machine, or a self-service machine, etc., which is not specifically limited in the embodiments of the present disclosure.

The apparatus in the embodiment of the present disclosure may be a device with an operating system. The operating system may be an Android operating system, an iOS operating system, or other possible operating systems, which are not specifically limited in the embodiment of the present disclosure.

The apparatus provided in the embodiment of the present disclosure can implement various processes implemented by the method embodiment shown in the figures, which is not repeated here.

FIG. 7 is a structural block diagram of an electronic device provided in an embodiment of the present disclosure.

As shown in FIG. 7, an embodiment of the present disclosure also provides an electronic device M00, including a processor M01, a memory M02, and a program or instruction stored in the memory M02 that, when executed by the processor M01, implements the various processes of the above-mentioned method embodiment. The same technical effect can be achieved, which is not repeated here.

It should be noted that the electronic device provided in the embodiments of the present disclosure includes the mobile electronic devices and non-mobile electronic devices mentioned above.

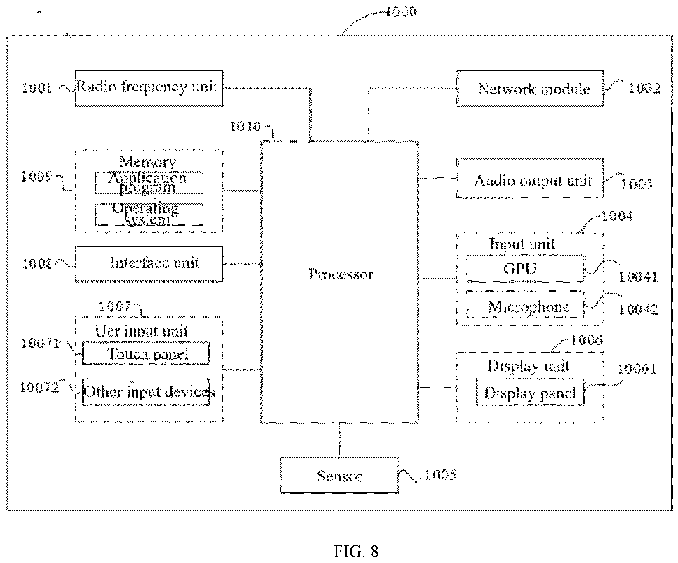

FIG. 8 is a schematic diagram of the hardware structure of an electronic device implementing an embodiment of the present disclosure.

The electronic device 1000 includes but is not limited to: a radio frequency unit 1001, a network module 1002, an audio output unit 1003, an input unit 1004, a sensor 1005, a display unit 1006, a user input unit 1007, an interface unit 1008, a memory 1009, and a C 1010.

Those skilled in the art will appreciate that the electronic device 1000 may also include a power source (such as a battery) for supplying power to various components, and the power source may be logically connected to the processor 1010 through a power management system, thereby implementing functions such as managing charging, discharging, and power consumption management through the power management system. The electronic device structure shown in FIG. 8 does not constitute a limitation on the electronic device. The electronic device may include more or fewer components than shown in the figure, or a combination of certain components, or a different arrangement of components, which will not be described in detail here.

It should be understood that in the embodiment of the present disclosure, the input unit 1004 may include a graphics processing unit (GPU) 10041 and a microphone 10042. The graphics processing unit 10041 is configured to processes image data of a still picture or a video obtained by an image capture device (such as a camera) in a video capture mode or an image capture mode. The display unit 1006 may include a display panel 10061, and the display panel 10061 may be configured in the form of a liquid crystal display, an organic light emitting diode, or the like. The user input unit 1007 includes a touch panel 10071 and other input devices 10072. The touch panel 10071 is also called a touch screen. The touch panel 10071 may include two parts, that is, a touch detection device and a touch controller. Other input devices 10072 may include, but are not limited to, a physical keyboard, function keys (such as a volume control key, a switch key, etc.), a trackball, a mouse, and a joystick, which will not be described in detail here. The memory 1009 may be used to store software programs and various data, including but not limited to application programs and operating systems. The processor 1010 may integrate an application processor and a modem processor, among them, the application processor mainly processes the operating system, user interface, and application programs, and the modem processor mainly processes wireless communications. It is understandable that the modem processor may not be integrated into the processor 1010.

An embodiment of the present disclosure also provides a readable storage medium, on which a program or instruction is stored. When the program or instruction is executed by a processor, the various processes of the above-mentioned method embodiment are implemented and the same technical effect can be achieved. To avoid repetition, it will not be repeated here.

The processor is the processor in the electronic device described in the above embodiment. The readable storage medium includes a computer readable storage medium, such as a computer read-only memory (ROM), a random access memory (RAM), a magnetic disk or an optical disk.

An embodiment of the present disclosure also provides a chip, including a processor and a communication interface. The communication interface is coupled to the processor, and the processor is used to run programs or instructions to implement the various processes of the above-mentioned method embodiment, and can achieve the same technical effect. To avoid repetition, it will not be repeated here.

It should be understood that the chip mentioned in the embodiments of the present disclosure can also be called a system level chip, a system chip, a chip system or a system-on-chip chip, etc.

Finally, it should also be noted that relational terms such as "first", "second" as used herein are merely used to distinguish an object or operation from another object or operation, and are not necessarily used to describe or imply that such an accrual relationship or sequence exists between these objects and operations. Furthermore, the terms "comprising" and "having", as well as any variations thereof, are intended to cover a non-exclusive inclusion, e.g. a process, method, item or terminal device including a series of steps or elements is not necessarily limited to those elements explicitly listed, but may include other elements not explicitly listed or inherent to the process, method, item or terminal device. In the absence of further restrictions, the elements limited by the phrase 'including a...' do not preclude the existence of additional identical elements in the process, method, item, or terminal device that includes the said element."

**Claims**

1. A computer-implemented method for determining a respiratory phase, applied to a ventilation therapy device comprising a respiratory conduit, wherein the method comprises:

   acquiring a gas flow value of the respiratory conduit, and acquiring a pressure value of a pressure sensor (101), wherein the pressure sensor is used for collecting a pressure in the respiratory conduit;
   converting the gas flow value and the pressure value into a first value and a second value under a same reference (102); and
   determining a current respiratory phase of a patient based on a difference of the first value and the second value (103).

2. The method according to claim 1, wherein the converting the gas flow value and the pressure value into a first value and a second value under a same reference (102) comprises:
   converting the gas flow value and the pressure value into a first value and a second value under a same physical quantity.

3. The method according to claim 2, wherein the converting the gas flow value and the pressure value into a first value and a second value under a same physical quantity comprises:
   converting the gas flow value into a first value under a pressure physical quantity, and using the pressure value as the second value.

4. The method according to claim 3, wherein the converting the gas flow value into a first value under a pressure physical quantity comprises:
   obtaining the first value under the pressure physical quantity by multiplying a square of the gas flow value by a drag coefficient of the ventilation therapy device.

5. The method according to claim 2 or 3, wherein the converting the gas flow value and the pressure value into a first value and a second value under a same physical quantity comprises:
   converting the pressure value into a second value under a flow physical quantity, and using the gas flow value as the first value.

6. The method according to claim 5, wherein the converting the pressure value into a second value under a flow physical quantity comprises:

   the pressure value being divided by a drag coefficient of the ventilation therapy device to obtain an intermediate value; and
   obtaining the second value under the flow physical quantity by finding a square root of the intermediate value.

7. The method according to claim 1 or 2, wherein the converting the gas flow value and the pressure value into a first value and a second value under a same reference (102) comprises:

   scaling the gas flow value and using the scaled gas flow value as the first value, and using the pressure value as the second value; or
   scaling the pressure value and using the scaled pressure value as the second value, and using the gas flow value as the first value.

8. The method according to claim 1, wherein the determining a current respiratory phase of a patient based on a difference of the first value and the second value (103) comprises:

   obtaining a first difference by subtracting the second value from the first value; and
   determining the current respiratory phase of the patient based on the first difference.

9. The method according to claim 8, wherein the determining the current respiratory phase of the patient based on the first difference comprises:

   determining the respiratory phase as an inspiratory phase in response to the first difference being greater than 0;
   determining the respiratory phase as an expiratory phase in response to the first difference being less than 0.

10. An apparatus for determining a respiratory phase, applied to a ventilation therapy device comprising a respiratory conduit, wherein the apparatus comprises:

   an acquisition module, configured to acquire a gas flow value of a respiratory conduit, and acquire a pressure value of a pressure sensor, wherein the pressure sensor is used for collecting a pressure in the respiratory conduit;
   a conversion module, configured to convert the gas flow value and the pressure value into a first value and a second value under a same reference; and
   a determination module, configured to determine a current respiratory phase of a patient based on a difference of the first value and the second value.

11. An electronic device, comprising a processor, a memory, and a program or instruction stored in the memory and executable on the processor, wherein the program or instruction, when executed by the processor, implements the method according to any one of claims 1 to 9.

12. A readable storage medium storing a program or instruction that, when executed by a processor, implements the method according to any one of claims 1 to 9.

## Patentansprüche

1. Computerimplementiertes Verfahren zum Bestimmen einer Atemphase, welches auf ein Gerät zur Atemtherapie angewendet wird, welches eine Atemleitung umfasst, wobei das Verfahren umfasst:

   Erfassen eines Gasflusswerts der Atemleitung und Erfassen eines Druckwerts eines Drucksensors (101), wobei der Drucksensor zum Erfassen eines Drucks in der Atemleitung verwendet wird,
   Umwandeln des Gasflusswerts und des Druckwerts in einen ersten Wert und einen zweiten Wert unter derselben Referenz (102) und
   Bestimmen einer aktuellen Atemphase eines Patienten auf der Grundlage einer Differenz zwischen dem ersten Wert und dem zweiten Wert (103).

2. Verfahren gemäß Anspruch 1, wobei das Umwandeln des Gasflusswerts und des Druckwerts in einen ersten Wert und einen zweiten Wert unter derselben Referenz (102) umfasst:
   Umwandeln des Gasflusswerts und des Druckwerts in einen ersten Wert und einen zweiten Wert unter derselben physikalischen Größe.

3. Verfahren gemäß Anspruch 2, wobei das Umwandeln des Gasflusswerts und des Druckwerts in einen ersten Wert und einen zweiten Wert unter derselben physikalischen Größe umfasst:
   Umwandeln des Gasflusswerts in einen ersten Wert unter einer physikalischen Größe Druck und Verwenden des Druckwerts als zweiten Wert.

4. Verfahren gemäß Anspruch 3, wobei das Umwandeln des Gasflusswerts in einen ersten Wert unter einer physikalischen Größe Druck umfasst:
   Ermitteln des ersten Wertes unter der physikalischen Größe Druck durch Multiplizieren eines Quadrats des Gasflusswerts mit einem Widerstandskoeffizienten des Geräts zur Atemtherapie.

5. Verfahren gemäß Anspruch 2 oder 3, wobei das Umwandeln des Gasflusswertes und des Druckwertes in einen ersten Wert und einen zweiten Wert unter derselben physikalischen Größe umfasst:
   Umwandeln des Druckwertes in einen zweiten Wert unter einer physikalischen Größe Fluss und Verwenden des Gasflusswertes als ersten Wert.

6. Verfahren gemäß Anspruch 5, wobei das Umwandeln des Druckwerts in einen zweiten Wert unter einer physikalischen Größe Fluss umfasst:

   das Teilen des Druckwerts durch einen Widerstandskoeffizienten des Geräts zur Atemtherapie, um einen Zwischenwert zu erhalten, und das Erhalten des zweiten Werts unter der physikalischen Größe Fluss durch Berechnen einer Quadratwurzel des Zwischenwerts.

**7.** Verfahren gemäß Anspruch 1 oder 2, wobei das Umwandeln des Gasflusswerts und des Druckwerts in einen ersten Wert und einen zweiten Wert unter derselben Referenz (102) umfasst:

Skalieren des Gasflusswerts und Verwenden des skalierten Gasflusswerts als ersten Wert und Verwenden des Druckwerts als zweiten Wert oder
Skalieren des Druckwerts und Verwenden des skalierten Druckwerts als zweiten Wert und Verwenden des Gasflusswerts als ersten Wert.

**8.** Verfahren gemäß Anspruch 1, wobei das Bestimmen einer aktuellen Atemphase eines Patienten auf der Grundlage einer Differenz zwischen dem ersten Wert und dem zweiten Wert (103) umfasst:

Ermitteln einer ersten Differenz durch Subtrahieren des zweiten Wertes vom ersten Wert und Bestimmen der aktuellen Atemphase des Patienten auf der Grundlage der ersten Differenz.

**9.** Verfahren gemäß Anspruch 8, wobei das Bestimmen der aktuellen Atemphase des Patienten auf der Grundlage der ersten Differenz umfasst:

Bestimmen der Atemphase als Inspirationsphase als Reaktion darauf, dass die erste Differenz größer als 0 ist,
Bestimmen der Atemphase als Exspirationsphase als Reaktion darauf, dass, die erste Differenz kleiner als 0 ist.

**10.** Vorrichtung zum Bestimmen einer Atemphase, welche an einem Gerät zur Atemtherapie, das eine Atemleitung umfasst, angewendet wird, wobei die Vorrichtung umfasst:

ein Erfassungsmodul, das so konfiguriert ist, dass es einen Gasflusswert einer Atemleitung erfasst und einen Druckwert eines Drucksensors erfasst, wobei der Drucksensor zum Erfassen eines Drucks in der Atemleitung verwendet wird,
ein Umwandlungsmodul, das so konfiguriert ist, dass es den Gasflusswert und den Druckwert unter derselben Referenz in einen ersten Wert und einen zweiten Wert umwandelt, und
ein Bestimmungsmodul, das so konfiguriert ist, dass es eine aktuelle Atemphase eines Patienten auf der Grundlage einer Differenz zwischen dem ersten Wert und dem zweiten Wert bestimmt.

**11.** Elektronisches Gerät, das einen Prozessor, einen Speicher und ein Programm oder eine Anweisung umfasst, welche in dem Speicher gespeichert und auf dem Prozessor ausführbar ist, wobei das Programm oder die Anweisung, wenn es/sie von dem Prozessor ausgeführt wird, das Verfahren gemäß einem der Ansprüche 1 bis 9 implementiert.

**12.** Lesbares Speichermedium, das ein Programm oder eine Anweisung speichert, welche, wenn sie von einem Prozessor ausgeführt wird, das Verfahren gemäß einem der Ansprüche 1 bis 9 implementiert.

**Revendications**

**1.** Procédé mis en œuvre par ordinateur de détermination d'une phase respiratoire, appliqué à un dispositif de thérapie par ventilation comprenant un conduit respiratoire, dans lequel le procédé comprend les étapes consistant à :

acquérir une valeur de flux de gaz du conduit respiratoire, et à acquérir une valeur de pression d'un capteur de pression (101), dans lequel le capteur de pression est utilisé pour collecter une pression dans le conduit respiratoire ;
convertir la valeur de flux de gaz et la valeur de pression en une première valeur et une seconde valeur sous une même référence (102) ; et
déterminer une phase respiratoire présente d'un patient sur la base d'une différence de la première valeur et de la seconde valeur (103).

**2.** Procédé selon la revendication 1, dans lequel la conversion de la valeur de flux de gaz et de la valeur de pression en une première valeur et une seconde valeur sous une même référence (102) comprend l'étape consistant à :
convertir la valeur de flux de gaz et la valeur de pression en une première valeur et une seconde valeur sous une même quantité physique.

**3.** Procédé selon la revendication 2, dans lequel la conversion de la valeur de flux de gaz et de la valeur de pression en une première valeur et une seconde valeur sous une même quantité physique comprend l'étape consistant à :
convertir la valeur de flux de gaz en une première valeur sous une quantité physique de pression, et à utiliser la valeur de pression comme seconde valeur.

**4.** Procédé selon la revendication 3, dans lequel la conversion de la valeur de flux de gaz en une première valeur sous une quantité physique de pression comprend l'étape consistant à :
obtenir la première valeur sous la quantité physique de pression en multipliant un carré de la valeur de flux de gaz par un coefficient de résistance du dispositif de thérapie par ventilation.

**5.** Procédé selon la revendication 2 ou 3, dans lequel la conversion de la valeur de flux de gaz et de la valeur de pression en une première valeur et une seconde valeur sous une même quantité physique comprend l'étape consistant à :
convertir la valeur de pression en une seconde valeur sous une quantité physique de flux, et à utiliser la valeur de flux de gaz comme première valeur.

**6.** Procédé selon la revendication 5, dans lequel la conversion de la valeur de pression en une seconde valeur sous une quantité physique de flux comprend :

la valeur de pression étant divisée par un coefficient de résistance du dispositif de thérapie par ventilation pour obtenir une valeur intermédiaire ; et
l'obtention de la seconde valeur sous la quantité physique de flux en trouvant une racine carrée de la valeur intermédiaire.

**7.** Procédé selon la revendication 1 ou 2, dans lequel la conversion de la valeur de flux de gaz et de la valeur de pression en une première valeur et une seconde valeur sous une même référence (102) comprend les étapes consistant à :

mettre à l'échelle la valeur de flux de gaz et à utiliser la valeur de flux de gaz mise à l'échelle comme première valeur, et à utiliser la valeur de pression comme seconde valeur ; ou
à mettre à l'échelle la valeur de pression et à utiliser la valeur de pression mise à l'échelle comme seconde valeur, et à utiliser la valeur de flux de gaz comme première valeur.

**8.** Procédé selon la revendication 1, dans lequel la détermination d'une phase respiratoire présente d'un patient sur la base d'une différence de la première valeur et de la seconde valeur (103) comprend les étapes consistant à :

obtenir une première différence par soustraction de la seconde valeur de la première valeur ; et
à déterminer la phase respiratoire présente du patient sur la base de la première différence.

**9.** Procédé selon la revendication 8, dans lequel la détermination de la phase respiratoire présente du patient sur la base de la première différence comprend les étapes consistant à :

déterminer la phase respiratoire comme phase inspiratoire en réponse à la première différence étant supérieure à 0 ;
déterminer la phase respiratoire comme phase expiratoire en réponse à la première différence étant inférieure à 0.

**10.** Appareil de détermination d'une phase respiratoire, appliqué à un dispositif de thérapie par ventilation comprenant un conduit respiratoire, dans lequel l'appareil comprend :

un module d'acquisition, configuré pour acquérir une valeur de flux de gaz d'un conduit respiratoire, et pour acquérir une valeur de pression d'un capteur de pression, dans lequel le capteur de pression est utilisé pour collecter une pression dans le conduit respiratoire ;
un module de conversion, configuré pour convertir la valeur de flux de gaz et la valeur de pression en une première valeur et une seconde valeur sous une même référence ; et
un module de détermination, configuré pour déterminer une phase respiratoire présente d'un patient sur la base d'une différence de la première valeur et de la seconde valeur.

**11.** Dispositif électronique, comprenant un processeur, une mémoire, et un programme ou une instruction stocké·e dans la mémoire et exécutable sur le processeur, dans lequel le programme ou l'instruction, lorsqu'exécuté·e par le processeur, met en œuvre le procédé selon l'une quelconque des revendications 1 à 9.

**12.** Support de stockage lisible stockant un programme ou une instruction qui, lorsqu'exécuté·e par un processeur, met en œuvre le procédé selon l'une quelconque des revendications 1 à 9.

Acquire a gas flow value of the respiratory conduit, and acquire a pressure value of a pressure sensor — 101

Convert the gas flow value and the pressure value into a first value and a second value under a same reference — 102

Determine a current respiratory phase of a patient based on a difference of the first value and the second value. — 103

FIG. 1

Senser signals

Gas flow value

Pressure value

Time

Patient's respiration

FIG. 2

FIG. 3

FIG. 4

FIG. 5

FIG. 6

FIG. 7

FIG. 8

**EP 4 427 779 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2021379306 A1